# EUROPÄISCHE PATENTSCHRIFT

(11) **EP 3 037 402 B1**
(45) Veröffentlichungstag und Bekanntmachung des Hinweises auf die Patenterteilung: **17.05.2017**
(21) Anmeldenummer: 15201229.0
(22) Anmeldetag: 18.12.2015
(51) Int. Cl.: C07C 67/00, C07C 69/24, C07C 69/34, C07C 69/42, C07C 69/612, C07C 69/65, C07C 69/732, C07D 209/48, C07D 307/83

(54) **VERFAHREN ZUR ALKOXYCARBONYLIERUNG VON ETHYLENISCH UNGESÄTTIGTEN VERBINDUNGEN**
METHOD FOR THE ALKOXYCARBONYLATION OF ETHYLENICALLY UNSATURATED COMPOUNDS
PROCÉDÉ D'ALKOXY-CARBONYLATION DE COMPOSÉS INSATURÉS EN ÉTHYLÈNE

(30) Priorität: 22.12.2014 DE 102014226870
(43) Veröffentlichungstag der Anmeldung: 29.06.2016
(73) Patentinhaber: Evonik Degussa GmbH, 45128 Essen (DE)
(72) Erfinder: BELLER, Matthias, 18211 Ostseebad Nienhagen (DE); JACKSTELL, Ralf, 27478 Cuxhaven Altenwalde (DE); LIU, Qiang, 300161 Hedong District Tianjin (CN); AROCKIAM, Percia Beatrice, 208 016 Kanpur Uttar Pradesh (IN); YUAN, Kedong, NanKai district 300384 TianJin (CN)

(56) Entgegenhaltungen:
- QIANG LIU ET AL: "Ruthenium-Catalyzed Alkoxycarbonylation of Alkenes with Paraformaldehyde as a Carbon Monoxide Substitute", CHEMCATCHEM, Bd. 6, Nr. 10, 2. September 2014 (2014-09-02), Seiten 2805-2809, XP055220035, DE ISSN: 1867-3880, DOI: 10.1002/cctc.201402304
- QIANG LIU ET AL: "Regioselective Pd-Catalyzed Methoxycarbonylation of Alkenes Using both Paraformaldehyde and Methanol as CO Surrogates", ANGEWANDTE CHEMIE INTERNATIONAL EDITION, Bd. 54, Nr. 15, 7. April 2015 (2015-04-07) , Seiten 4493-4497, XP055219963, DE ISSN: 1433-7851, DOI: 10.1002/anie.201410764

## Beschreibung

Die Erfindung betrifft die Alkoxycarbonylierung von ethylenisch ungesättigten Verbindungen, wobei mindestens eine ethylenisch ungesättigte Verbindung mit Formalin oder Paraformaldehyd und einem Alkohol ausgewählt aus Methanol oder Isopropanol in Gegenwart eines Palladiumkatalysators und einer Säure als Cokatalysator umgesetzt wird. Übergangsmetall-katalysierte Carbonylierungsreaktionen sind ein leistungsfähige Werkzeuge sowohl in der organischen Synthese als auch in der industriellen Produktion verschiedener Carbonylverbindungen, wie Ester, Amide, Ketone und Aldehyde, etc. Unter Verwendung von Palladiumkatalysatoren ist insbesondere die Methoxycarbonylierung von Bedeutung (C. Jimenez Rodriguez, D. F. Foster, G. R. Eastham, D. J. Cole-Hamilton, Chemical Communications 2004, 0, 1720-1721). Ein großer Nachteil des Verfahrens ist die Verwendung des Gases Kohlenmonoxid, das toxisch ist sowie leicht entflammbar und deshalb auch schwierig in großen Mengen zu transportieren. In den letzten Jahren wurden beträchtliche Anstrengungen für die Entwicklung neuartiger carbonylierender Transformationen mit CO Alternativen unternommen. Hierbei kann das toxische CO-Gas durch bequemere anorganische oder organische Carbonylverbindungen ersetzt werden.

So ist der z.B. der Einsatz von Formiaten als CO Erzatzquellen in katalytischen Alkoxycarbonylierungen bekannt (H. Li, H. Neumann, M. Beller, X.-F. Wu, Angew. Chem. Int. Ed. 2014, 53, 3183-3186.).

Formiate sind bekanntermaßen ein Addukt aus Kohlenmonoxid und Alkohol. Es wird bevorzugt Methylformiat verwendet, welches industriell aus Methanol, Kohlenmonoxid und einer starken Base hergestellt wird. Dementsprechend ist die Carbonylierung mit Methylformiat nicht wirklich eine kohlenmonoxidfreie Reaktion. Q. Liu, L. Wu, R. Jackstell, M. Beller, ChemCatChem 2014, 6, 2805-2809 zeigt die Herstellung von Estern durch Umsetzung von Olefinen mit Paraformaldehyd und Alkoholen in Gegenwart eines Rutheniumkatalysators.

Die Aufgabe der Erfindung besteht daher darin, ein einfaches Verfahren zur katalytischen Alkoxycarbonylierung bereitzustellen, das es ermöglicht, insbesondere Methylester in guten Ausbeuten kostengünstig herzustellen, wobei der Einsatz von Kohlenmonoxid vermieden werden soll. Ein besonderes Ziel ist es, auch langkettige Alkene der Carbonylierung zugänglich zu machen. Dabei sollen gute Regioselektivitäten (>90%) und gute Aktivitäten (TOF >100h⁻¹) erreicht werden.

Es wurde gefunden, dass diese Aufgabe durch homogene Katalyse mittels eines katalytischen Systems auf Palladiumbasis gelöst werden kann. Erfindungsgemäß wird mindestens eine ethylenisch ungesättigte Verbindung mit Formalin oder Paraformaldehyd und einem Alkohol ausgewählt aus Methanol und Isopropanol in Gegenwart eines Palladiumkatalysators, mindestens einem Phosphinliganden und einer Säure umgesetzt.

Paraformaldehyd besitzt die allgemeine Formel [CH₂O]ₙ, wobei n = 1 bis 200 sein kann. Als Paraformaldehyd wird das feste Formaldehyd-Polymer bezeichnet, das eine Mischung von kurzkettigen, linearen Poly(oxymethylen)en enthält und das z.B. beim Eindampfen von Formaldehyd-Lösung im Vakuum erhältlich ist. Beim Erhitzen auf ca. 100 °C zerfällt Paraformaldehyd reversibel oder durch Säure-Einwirkung in monomeres Formaldehyd. Neben Paraformaldehyd ist Formalin (etwa 35-37 %ige wässrige Lösung des Formaldehyd-Gases, wobei 10-15 Gew.-% Methanol zur Stabilisierung gegen Polymerbildung höherer Aggregate zugesetzt sind) im erfindungsgemäßen Verfahren geeignet.

Erfindungsgemäß werden Formalin oder Paraformaldehyd sowie die Alkohole Methanol oder Isopropanol vorzugsweise im Überschuss eingesetzt.

Ethylenisch ungesättigte Verbindungen, die im erfindungsgemäßen Verfahren als Ausgangsmaterialien fungieren, können einfach oder mehrfach ungesättigte lineare n-Alkene oder verzweigte Isoalkene, Cycloalkene und aromatische Alkene mit einer Kohlenstoffzahl bevorzugt von 2 bis 40 sein, die gegebenenfalls substituiert sind. Vorzugsweise werden Alkene mit 2 bis zu 20 C-Atomen eingesetzt.

Als Substituenten kommen Ester-, Aryl-, Nitro-, Hydroxyl-, Nitril-, Ethergruppen oder Halogenatome in Frage.

Beispielsweise seien Ethen, Propylen, Buten, Isobuten, Penten, Hexen, Hepten, 1- und 2-Octen, Octa-1,7-dien, Cyclopenten, Cyclohexen, Bicyclo[2.2.1]hept-2-en, Styren, Allylbenzol, Isopropenylbenzol, 1-Isopropenyl-4-methylbenzol, 1-Chlor- 4-isopropenylbenzol, 1-Fluor-4-isopropenylbenzol, 2-Isopropenylnaphthalin, Methylacrylat, Methylmethacrylat genannt. Weiterhin sind auch Gemische verschiedener ethylenisch ungesättigter Verbindungen einsetzbar.

Der beim erfindungsgemäßen Verfahren verwendete Palladiumkatalysator wird bevorzugt in Form einer Vorläuferverbindung als Palladiumkomplexverbindung eingesetzt, die durch den Phosphinliganden koordiniert wird. In einer bevorzugten Ausführungsvariante des Verfahrens weist der Katalysator mindestens einen Palladiumkomplex mit Palladium in den Oxidationsstufen 0 bis +II auf. Beispielsweise seien die Palladiumkomplexe Pd(II)-Acetat, Pd(II)-Acetylacetonat, Pd(II)-Halogenide (Pd(II)(cod)Cl₂, Pd(MeCN)₂Cl₂) und Pd(II)-Dibenzylidenaceton genannt.

Der Phosphinligand weist bevorzugt eine bidentate Struktur auf. Als besonders geeignet hat sich im erfindungsgemäßen Verfahren der Phosphinligand 1,2-Bis(di-*tert-*butylphosphinomethyl)benzol (d^{t}bpx) gezeigt. Die Liganden können gegebenenfalls in einer Vorreaktion mit dem Palladiumkomplex kombiniert werden oder sie werden in situ zur Reaktion gegeben. Ganz besonders bevorzugt wird im erfindungsgemäßen Verfahren ein Katalysator umfassend Pd-Acetat und d^{t}bpx verwendet.

Die ethylenisch ungesättigten Verbindungen werden vorzugsweise mit der wenigstens einfachen äquivalenten Menge des jeweiligen Alkohols und des Formaldehyds bzw. Formalins in Gegenwart des Pd-Katalysators umgesetzt. Die Umsetzung führt zu den gewünschten Methyl- oder Isopropylestern in Abhängigkeit von der verwendeten ethylenisch ungesättigten Verbindung.

Die Säure ist bevorzugt eine Sulfon- oder eine Phosphinsäure, besonders bevorzugt sid Methansulfonsäure oder Toluolsulfonsäuren oder Diarylphosphinsäuren. Erfindungsgemäß liegt der Phosphinligand vorzugsweise in einer Menge vor, die unterhalb der Menge an Sulfonsäure liegt oder die der Menge an Sulfonsäure entspricht.

Der Palladiumkatalysator umfasst den Phosphinliganden und die Sulfonsäure im Verhältnis Palladium : Ligand im Bereich von 1 : 2 bis 1 :20 und Palladium : Säure im Bereich von 1 : 3 bis 1: 30. Alle Verhältnisse sind Molverhältnisse.

Wirksame Katalysatormengen im Verfahren liegen vorzugsweise bei 0,01 bis 12 Mol-% bezogen auf die eingesetzte ethylenisch ungesättigte Verbindung. Die molaren Verhältnisse ethylenisch ungesättigte Verbindung : Methanol oder Isopropanol können von 1: 75 bis 1:1 variieren. Das Verhältnis von ethylenisch ungesättigter Verbindung zu Formaldehyd liegt z.B. bei 1 : 100, bevorzugt 1 : 10, besonders bevorzugt 1 : 4.

Im erfindungsgemäßen Verfahren erfolgt die Reaktion vorzugsweise bei Temperaturen von 20 bis 160°C, besonders bevorzugt bei Temperaturen von 80 bis 140°C. Es muss nicht unter besonderen Druckbedingungen gearbeitet werden. Der Druckbereich liegt vorzugsweise bei Drücken von 1 bis 60 bar, besonders bevorzugt bei Normaldruck der Reaktionsmischung bei Reaktionstemperatur.

Die Reaktionsdauer hängt von der gewählten Temperatur ab. Sie liegt für die weitgehendste Umsetzung der ethylenisch ungesättigten Verbindung bevorzugt bei ca. 1 bis 36 Stunden.

Das erfindungsgemäße Verfahren ermöglicht auf einfache Weise kostengünstig insbesondere die Herstellung beliebiger gesättigter Methylesterverbindungen. Die Reaktion kann in hohen Ausbeuten und mit hoher Selektivität durchgeführt werden. Eine gesonderte Aufarbeitung unter Entfernung von Nebenprodukten kann entfallen.

Im Vergleich zu Formiaten kann das erfindungsgemäß eingesetzte Formaldehyd technisch durch die katalytische Oxidation von Methanol, das über CO₂ Hydrierung in großem Maßstab produziert wird, erhalten werden. Außerdem bietet die Nutzung von Formalin bzw. Paraformaldehyd die Möglichkeit, die Verwendung von kostenintensiven Hochdruckapparaturen vollständig zu vermeiden.

Die Erfindung wird in nachfolgenden Beispielen näher erläutert.

### Allgemeine Betrachtungen

Alle kommerziellen Chemikalien wurden bei Alfa Aesar, Aldrich oder Strem georderd. Falls nicht anders vermerkt wurden sie ohne weitere Aufreinigung verwendet. Luft- und feuchtigkeitsempfindliche Substanzen wurden unter Argon in getrockneten Glasapparaturen verwendet. Analytische Daten der literaturbekannten Verbindungen waren in Einklang mit gefundenen Daten. Die NMR Spektren wurden mit einem Bruker Avance 300 (300 MHz) und Bruker Avance 400 (400 MHz) NMR Spektrometer aufgenommen. Die chemischen Verschiebungen δ (ppm) sind relativ zum Lösungsmittel angegeben mit folgenden Referenzen: für CDCl₃ 7.26 ppm (¹H-NMR) und 77.16 ppm (¹³C-NMR). ¹³C-NMR Spektren wurden im Breitbandentkopplungsmodus gemessen. Multipletts wurden wie folgt abgekürzt. S als Singulett, t als Triplett, dd als Dublett eines Dubletts, d als Dublett, m als Multplett und br. s als breites Singulett. Alle Messungen wurden falls nicht anders angegeben bei Raumtemperaur gemessen.. Elektronenstoß (El) Massenspektren wurden auf einem AMD 402-Massenspektrometer (70 eV) aufgenommen. Hochauflösende Massenspektren (HRMS) wurden auf mit einem Agilent 6210 gemesssen, Time-of-Flight LC/MS (Agilent) mit Elektrospray-Ionisation (ESI). Die Daten werden als Masseeinheiten pro Ladung (m / z) und die Intensitäten von Signalen in Klammern angegeben. Für GC-Analysen, sind HP 6890 Chromatographen mit einer 29 m HP5-Säule verwendet worden.

### Experimetalbeispiele

### Abkürzungsverzeichnis

### dtbpx - 1,2-Bis((di-tert-butylphosphanyl)methyl)benzen

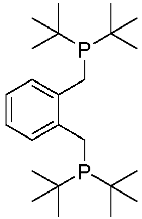

### PTSA- Paratoluolsulfonsäure

**Tabelle 1. Pd-Katalysierte Methoxycarbonylierung von 1-Octen 1a.^{[a]}**

| 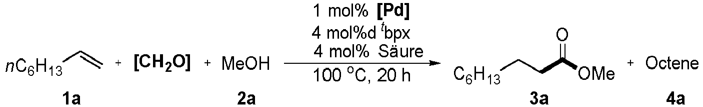 | | | | | |
|---|---|---|---|---|---|
| Eintrag | [CH₂O] | [Pd] | Säure | Ausbeute **3a** (I :b) | Ausbeute **4a** |
| 1 | (CH₂O) ₙ | Pd(acac)₂ | PTSA | 74% (95:5) | 25% |
| 2 | (CH₂O) ₙ | Pd(acac)₂ | MSA | 74% (95:5) | 24% |
| 3 | Formali ₙ | Pd(acac)₂ | PTSA | 56% (95:5) | 11% |
| 4 | (CH₂O)ₙ | Pd(OAc)₂ | PTSA | 78% (95:5) | 22% |
| 5 | (CH₂O) ₙ | Pd(cod)Cl₂ | PTSA | 10% (91:9) | 90% |
| 6 | (CH₂O) ₙ | Pd(MeCN) ₂Cl₂ | PTSA | 10% (97:3) | 90% |
| 7 | (CH₂O) ₙ | Pd₂(dba)₃ | PTSA | 16% (96:4) | 82% |

| | | | | | |
|---|---|---|---|---|---|
| [a] Reaktionsbedingungen: **1a** (1 mmol), 'CH₂O' Monomer (6.7 mmol), [Pd] (1 mol%), d*^{t}*bpx (4 mol%), Säure (4 mol%) in 2 mL Methanol **2a** bei 100 °C für 20 h. Ausbeutebestimmung mittels GC Analyse. d*^{t}*bpx = α,α'-Bis-[di-tert-butylphosphino]-o-xylen, PTSA = *p*-Toluolsulfonsäure, MSA = Methansulfonsäure. | | | | | |

### Beispiel 1 (Tabelle 1, Eintrag 1):

Unter einer Schutzgasatmosphäre von Argon wird ein verschließbares 25 ml Glasrohr, versehen mit einem Magnetrührer mit Pd(acac)₂ (3 mg, 0.01 mmol), d*^{t}*bpx (15,8 mg, 0.04 mmol), PTSA·H₂O (7,6 mg, 0,04 mmol) und Paraformaldehyd (200 mg, 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 74 % mit einer n-Selektivität von 95 %.

### Beispiel 2 (Tabelle 1, Eintrag 2):

Unter einer Schutzgasatmosphäre von Argon wird ein verschließbares 25 ml Glasrohr, versehen mit einem Magnetrührer mit Pd(acac)₂ (3 mg, 0.01 mmol), d*^{t}*bpx (15,8 mg, 0.04 mmol), MSA(Methansulfonsäure, (3,86 mg, 0,0026 ml,0,04 mmol) und Paraformaldehyd (200 mg, 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 74 % mit einer n-Selektivität von 95 %.

### Beispiel 3 (Tabelle 1, Eintrag 3):

Unter einer Schutzgasatmosphäre von Argon wird ein verschließbares 25 ml Glasrohr, versehen mit einem Magnetrührer mit Pd(acac)₂ (3 mg, 0.01 mmol), d*^{t}*bpx (15,8 mg, 0.04 mmol), PTSA·H₂O (7,6 mg, 0,04 mmol) und Formalin (0,55ml, 6,7 mmol-CH2O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysier.t Die Ausbeute an Methylnonanoaten **3a** beträgt 56 % mit einer n-Selektivität von 95 %.

### Beispiel 4 (Tabelle 1, Eintrag 4):

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA-H₂O (7,6 mg, 0,04 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 78 % mit einer n-Selektivität von 95 %.

### Beispiel 5 (Tabelle 1, Eintrag 5):

Unter einer Schutzgasatmosphäre von Argon wird ein verschließbares 25 ml Glasrohr, versehen mit einem Magnetrührer mit Pd(COD)Cl₂ (2,85 mg, 0.01 mmol), d*^{t}*bpx (15,8 mg, 0.04 mmol), PTSA·H₂O (7,6 mg, 0,04 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 10 % mit einer n-Selektivität von 91 %.

### Beispiel 6 (Tabelle 1, Eintrag 6):

Unter einer Schutzgasatmosphäre von Argon wird ein verschließbares 25 ml Glasrohr, versehen mit einem Magnetrührer mit Pd(CH₃CN)₂Cl₂ (2,59 mg, 0.01 mmol), d*^{t}*bpx (15,8 mg, 0.04 mmol), PTSA·H₂O (7,6 mg, 0,04 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 10 % mit einer n-Selektivität von 97 %.

### Beispiel 7 (Tabelle 1, Eintrag 7):

Unter einer Schutzgasatmosphäre von Argon wird ein verschließbares 25 ml Glasrohr, versehen mit einem Magnetrührer mit Pd₂(dba)₃ (4,5 mg, 0.005 mmol), d*^{t}*bpx (15,8 mg, 0.04 mmol), PTSA·H₂O (7,6 mg, 0,04 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 16 % mit einer n-Selektivität von 96 %.

**Tabelle 2 Liganden - und Cokatalysatoreffekte der Methoxycarbonylierung von 1a.[a]**

| 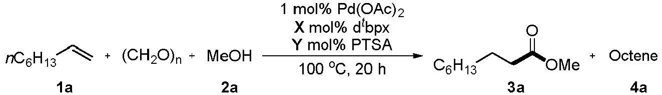 | | | | |
|---|---|---|---|---|
| Eintrag | X | Y | Ausbeute **3a** (I :b) | Ausbeute **4a** |
| 1 | 4 | 4 | 78% (95:5) | 22% |
| **2** | **4** | **5** | **96% (95:5)** | **4%** |
| 3 | 4 | 6 | 68% (95:5) | 38% |
| 4 | 4 | 8 | 64% (95:5) | 36% |
| 5 | 2 | 4 | 17% (96:4) | 74% |

| | | | | |
|---|---|---|---|---|
| [a] Reaktionsbedingungen: **1a** (1 mmol), (CH₂O)ₙ (200 mg, 6.7 mmol (CH₂O) Pd(OAc)₂ (1 mol%), d*^{t}*bpx (X mol%), PTSA(Y mol%) in 2 mL Methanol **2a** bei 100 °C für 20 h. Ausbeutebestimmung mittels GC Analyse | | | | |

### Beispiel 8 (Tabelle 2, Eintrag 1, Tabelle 1 Eintrag 4)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (7,6 mg, 0,04 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 78 % mit einer n-Selektivität von 95 %.

### Beispiel 9 (Tabelle 2, Eintrag 2)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 96 % mit einer n-Selektivität von 95 %.

### Beispiel 10 (Tabelle 2, Eintrag 3)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (11,4 mg, 0,06 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 68 % mit einer n-Selektivität von 95 %.

### Beispiel 11 (Tabelle 2, Eintrag 4)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (15,2 mg, 0,08 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 64 % mit einer n-Selektivität von 95 %.

### Beispiel 12 (Tabelle 2, Eintrag 5)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (7,9 mg, 0.02 mmol), PTSA·H₂O (7,6 mg, 0,04 mmol) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 17% mit einer n-Selektivität von 96%.

### Allgemeine Versuchsvorschrift zur palladiumkatalysiertem Methoxycarbonylierung von Alkenen 1 mit Paraformaldehyd und Methanol 2a.

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das Alken **1** (1 mmol) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus

Heptan und Ethylacetat gereinigt, und im Vakuum getrocknet. Es resultieren die Verbindungen **3a** bis **3o.**

**Tabelle 3. Pd-Katalysierte Methoxycarbonylierung von Alkenen 1 mit Paraformaldehyd und Methanol 2a.^{[a]}**

| | | | |
|---|---|---|---|
| 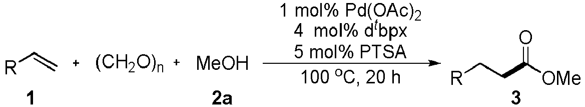 | | | |

| Eintrag | Substrat **1** | Produkt **3**^{[b]} | Ausbeute **3** (I :b)^{[c]} |
|---|---|---|---|
| 1 | 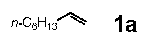 | 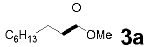 | 93% (95:5) |
| 2 | 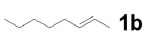 | 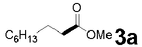 | 93% (94:6) |
| 3 | 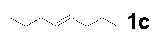 | 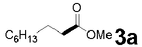 | 75% (89:11) |
| 4 | 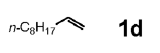 | 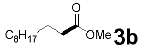 | 92% (95:5) |
| 5 | 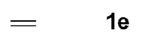 | 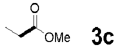 | 99% |
| 6 | 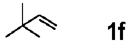 | 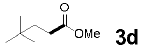 | 85%(> 99:1) |
| 7 | 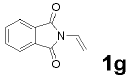 | 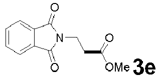 | 55% (>99:1) |
| 8 | 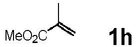 | 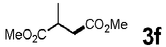 | 89% (> 99:1) |
| 9 | 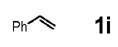 | 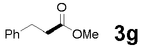 | 90% (78:22) |
| 10 | 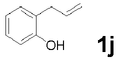 | 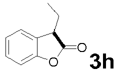 | 58%, 8 h^{[d]} |
| 11 | 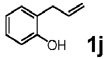 | 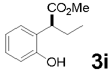 | 75%, 36 h^{[d]} |
| 12 | 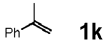 | 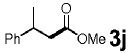 | 76% (> 99:1) |
| 13 | 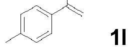 | 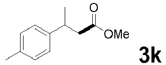 | 69% (> 99:1) |
| 14 | 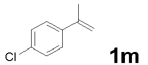 | 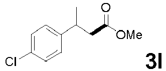 | 43% (> 99:1) |
| 15 | 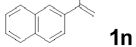 | 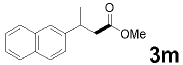 | 73% (> 99:1) |
| 16 | 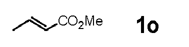 | 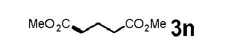 | 92% (> 99:1) |
| 17 | 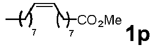 | 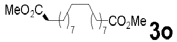 | 86% (94:6) |

| | | | |
|---|---|---|---|
| [a] Reaktionsbedingungen: 1 (1 mmol), (CH₂O)ₙ (200 mg, 6.7 mmol -CH₂O), Pd(OAc)₂ (1 mol%), d*^{t}*bpx (4 mol%), PTSA (5 mol%) in 2 mL Methanol **2a** bei 100 °C für 20 h. [b] Das Hauptisomer der Mischung verschiedener Methylester ist dargestellt. [c] Isolierte Ausbeute der Methylesterisomerenmischung . [d] Reaktionszeit. | | | |

### Beispiel 13 (Tabelle 3, Eintrag 1)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash -Chromatografie über Kieselgel 60 mit einer Mischung aus Heptan und Ethylacetat gereinigt, und im Vakuum getrocknet. Die isolierte Ausbeute an Methylnonanoaten **3a** beträgt 93 % mit einer n-Selektivität von 95%.

### Beispiel 14 (Tabelle 3, Eintrag 2)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 2-Octen **1b** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash -Chromatografie über Kieselgel 60 mit einer Mischung aus Heptan und Ethylacetat gereinigt, und im Vakuum getrocknet. Die isolierte Ausbeute an Methylnonanoaten **3a** beträgt 93 % mit einer n-Selektivität von 94%.

### Beispiel 15 (Tabelle 3, Eintrag 3)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das trans-4-Octen **1c** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methylnonanoaten **3a** beträgt 75 % mit einer n-Selektivität von 89%.

### Beispiel 16 (Tabelle 3, Eintrag 4)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 1-Decen **1d** (1 mmol, 140 mg, 0,19 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methylundecenoaten **3b** beträgt 85 % mit einer n-Selektivität von 95%.

### Beispiel 17 (Tabelle 3, Eintrag 5)

Unter Argonatmosphäre wird ein 25 ml Stahlautoklav mit Pd(OAc)₂ (2,2 mg, 0.01 mmol), d*^{t}*bpx (15,8 mg, 0.04 mmol), PTSA·H₂O (7,6 mg, 0,04 mmol) und Paraformaldehyd (1,2 g) befüllt. Anschließend werden 5 bar Ethylen **1e** aufgepreßt (150 mg, 5,4 mmol). Anschließend wird Methanol **2a** (6 mL) zugesetzt. Der Reaktor wird 20 h bei 100°C gerührt. Anschließend wird mittels eines Eisbades heruntergekühlt und Restdruck abgelassen. Die Reaktionsmischung wird mittels Gaschromatographie und Isooctan als internem Standard untersucht. Die Ausbeute an Methylpropionat **3c** beträgt mehr als 99%.

### Beispiel 18 (Tabelle 3, Eintrag 6)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 3,3-Dimethylbut-1-en **1f** (1 mmol, 84 mg, 0,129 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methyl-4,4-dimethylpentanoaten **3d** beträgt 85 % mit einer n-Selektivität von größer 99%.

### Beispiel 19 (Tabelle 3, Eintrag 7)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 2-Vinylisoindoline-1,3-dion **1g** (1 mmol, 173 mg) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methyl-3-(1,3-Dioxoisoindolin-2-yl)propanoat **3e** beträgt 55 % mit einer n-Selektivität von größer 99%.

### Beispiel 20 (Tabelle 3, Eintrag 8)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das Methylmethacrylat **1h** (1 mmol, 100 mg) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash -Chromatografie über Kieselgel 60 mit einer Mischung aus Heptan und Ethylacetat gereinigt, und im Vakuum getrocknet. Die isolierte Ausbeute an Dimethyl- 2-methylsuccinat **3f** beträgt 89 % mit einer n-Selektivität von größer 99%.

### Beispiel 21 (Tabelle 3, Eintrag 9)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das Styren **1i** (1 mmol, 104 mg) und MeOH **2a** (2 mL) zugegeben. Nach dem Verschließen des Glasrohres wird die Reaktionsmischung 36 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methyl 3-phenylpropanoat **3g** beträgt 90 % mit einer n-Selektivität von 78%.

### Beispiel 22 (Tabelle 3, Eintrag 10)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 2-Allylphenol **1j** (1 mmol, 134 mg) und MeOH **2a** (2 mL) zugegeben. Nach dem Verschließen des Glasrohres wird die Reaktionsmischung 8 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet..Die isolierte Ausbeute an 3-Ethylbenzofuran-2(3H)-on **3h** beträgt 58 %.

### Beispiel 23 (Tabelle 3, Eintrag 11)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 2-Allylphenol **1j** (1 mmol, 134 mg) und MeOH **2a** (2 mL) zugegeben. Nach dem Verschließen des Glasrohres wird die Reaktionsmischung 36 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methyl-2-(2-hydroxyphenyl)butanoat **3i** beträgt 75 %.

### Beispiel 24 (Tabelle 3, Eintrag 12)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das Prop-1-en-2-ylbenzen **1k** (1 mmol, 118 mg) und MeOH **2a** (2 mL) zugegeben. Nach dem Verschließen des Glasrohres wird die Reaktionsmischung 36 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash -Chromatografie über Kieselgel 60 mit einer Mischung aus Heptan und Ethylacetat gereinigt, und im Vakuum getrocknet. Die isolierte Ausbeute an Methyl-3-phenylbutanoat beträgt **3j** 76 % mit einer n-Selektivität von größer 99:1.

### Beispiel 25 (Tabelle 3, Eintrag 13)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 1-Methyl-4-(prop-1-en-2-yl)benzen **1l** (1 mmol, 132 mg) und MeOH **2a** (2 mL) zugegeben. Nach dem Verschließen des Glasrohres wird die Reaktionsmischung 36 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methyl 3-(p-tolyl)butanoat **3k** beträgt 69 % mit einer n-Selektivität von größer 99:1.

### Beispiel 26 (Tabelle 3, Eintrag 14)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 1-Chlor-4-(prop-1-en-2-yl)benzen **1m** (1 mmol, 152 mg) und MeOH **2a** (2 mL) zugegeben. Nach dem Verschließen des Glasrohres wird die Reaktionsmischung 36 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methyl 3-(4-chlorphenyl)butanoat **3l** beträgt 43 % mit einer n-Selektivität von größer 99:1.

### Beispiel 27 (Tabelle 3, Eintrag 15)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das 2-(Prop-1-en-2-yl)naphthalen **1n** (1 mmol, 168 mg) und MeOH **2a** (2 mL) zugegeben. Nach dem Verschließen des Glasrohres wird die Reaktionsmischung 36 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Methyl-3-(naphthalen-2-yl)butanoat **3m** beträgt 73% mit einer n-Selektivität von größer 99:1.

### Beispiel 28 (Tabelle 3, Eintrag 16)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das Methyl-(E)-but-2-enoat **1o** (1 mmol, 100 mg) und MeOH **2a** (2 mL) zugegeben. Nach dem Verschließen des Glasrohres wird die Reaktionsmischung 36 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Dimethylglutarat **3n** beträgt 92 % mit einer n-Selektivität von größer 99:1.

### Beispiel 29 (Tabelle 3, Eintrag 17)

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (15,8 mg, 0.04 mmol), PTSA·H₂O (9,5 mg, 0,05 mmol) und Paraformaldehyd (200 mg) eingefüllt. Anschließend wird das Methyloleat **1p** (1 mmol, 1296 mg) und MeOH **2a** (2 mL) zugegeben. Nach dem Verschließen des Glasrohres wird die Reaktionsmischung 36 Stunden bei 100 °C gerührt. Dann wird die Reaktionsmischung abgekühlt und mittels Gaschromatographie mit Isooctan als internen Standard analysiert. Hiernach werden 10 ml Wasser hinzugegeben. Diese Mischung wird dreimal mit 20 ml Ethylacetat extrahiert. Die vereinigten organischen Phasen werden mit wasserfreiem Natriumsulfat getrocknet und im Vakuum eingeengt. Dieser Rückstand wird mittels Flash-Chromatografie über Kieselgel 60 mit einer Mischung aus Heptan und Ethylacetat gereinigt, und im Vakuum getrocknet. Die isolierte Ausbeute an Dimethyl -1,19-nonadecanedioat **3o** beträgt 86 % mit einer n-Selektivität von 94%.

### Beispiel 30

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (7,9 mg, 0.02 mmol), **Benzolsulfonsäure** (8,3 mg) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 92% mit einer n-Selektivität von 95%.

### Beispiel 31

Unter einer Schutzgasatmosphäre von Argon wird ein verschließbares 25 ml Glasrohr, versehen mit einem Magnetrührer mit Pd(OAc)₂ (2,2 mg, 0.01 mmol), d*^{t}*bpx (7,9 mg, 0.02 mmol), **Diphenylphosphinsäure** (10,9 mg) und Paraformaldehyd (200 mg 6,7 mmol-CH₂O-) eingefüllt. Anschließend wird das 1-Octen **1a** (1 mmol, 112 mg, 0,157 ml) und MeOH **2a** (2 mL) zugegeben. Nach dem Verschließen des Glasrohres wird die Reaktionsmischung 20 Stunden bei 100 °C gerührt. Dann wird die Reaktionsmischung abgekühlt und mittels Gaschromatographie mit Isooctan als internen Standard analysiert. Die Ausbeute an Methylnonanoaten **3a** beträgt 7% mit einer n-Selektivität von 99%.

### Beispiel 32:

### Methoxycarbonylierung von 1-Octen 1 a mit Isopropanol 2c:

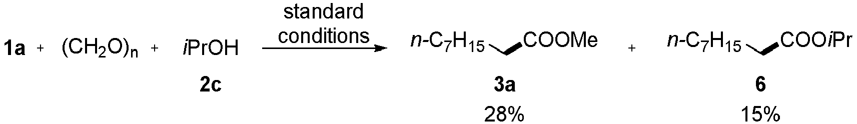

Unter Argon wird ein 25 ml verschließbares Glasrohr und Magnetrührer mit Pd(OAc)₂ (2,2 mg, 0.01 mmol), d*^{t}*bpx (15,8 mg, 0.04 mmol), PTSA·H₂O (7,6 mg, 0,04 mmol) und Paraformaldehyd (200 mg) befüllt. Anschließend werden 1-Octen **1a** (1 mmol, 112 mg 0,157 ml) und Isopropanol **2c** (2 mL) zugesetzt. Nach dem Verschließen des Glasgefäßes wird 20 h bei 100 °C gerührt. Dann wird das Reaktionsgemisch gaschromatogaphisch untersuchtz. Die Ausbeute an Methylnonanoat **3a** beträgt 28 % und an Isopropylnonanoat **6** 15%.

### Charakterisierung der Methylester 3.

**Methylnonanoat¹** (3a): ¹H NMR (300 MHz, CDCl₃) δ 3.60 (s, 3H), 2.23 (t, *J* = 7.5 Hz, 2H), 1.58-1.50 (m, 2H), 1.21 (br, 10H), 0.83-0.79 (m, 3H). ¹³C NMR (75 MHz, CDCl₃) δ 174.3, 77.4, 77.0, 76.6, 51.4, 34.1, 31.8, 29.2, 29.1, 25.0, 22.6, 14.1. HRMS (ESI) berechnet für C₁₀H₂₀O₂: 172.14578; gfunden: 172.14632.
**Methylundecanoat² (3b):** ¹H NMR (300 MHz, CDCl₃) δ 3.60 (s, 3H), 2.23 (t, *J* = 7.5 Hz, 2H), 1.57-1.52 (m, 2H), 1.19 (br, 14H), 0.83-0.79 (m, 3H). ¹³C NMR (75 MHz, CDCl₃) δ 174.3, 77.4, 77.0, 76.6, 51.4, 34.1, 31.8, 29.5, 29.4, 29.3, 29.2, 29.1, 25.0, 22.7, 14.1. HRMS (ESI) berechnet für C₁₂H₂₄O₂: 200.17708; gefunden: 200.17692.
**Methyl- 4,4-dimethylpentanoat³ (3d):** ¹H NMR (300 MHz, CDCl₃) δ 3.60 (s, 3H), 2.24-2.19 (m, 2H), 1.51-1.45 (m, 2H), 0.82 (s, 9H). ¹³C NMR (75 MHz, CDCl₃) δ 174.9, 77.4, 77.0, 76.6, 51.5, 38.6, 30.0, 29.9, 29.7, 29.0.
**Methyl- 3-(1,3-dioxoisoindolin-2-yl)propanoat⁴ (3e):** ¹H NMR (300 MHz, CDCl₃) δ 7.79-7.76 (m, 2H), 7.66-7.64 (m, 2H), 3.93 (t, *J* = 6.0 Hz, 2H), 3.61 (s, 3H), 2.67 (t, *J* = 7.5 Hz, 2H). ¹³C NMR (75 MHz, CDCl₃) δ 171.2, 170.0, 134.1, 132.0, 123.4, 77.5, 77.1, 76.6, 51.9, 33.8, 32.8. HRMS (ESI) berechnet für C₁₂H₁₁NO₄Na⁺: 256.05803; gefunden: 256.05808.
**Dimethyl- 2-methylsuccinat⁵ (3f):** ¹H NMR (300 MHz, CDCl₃) δ 3.74 (s, 3H), 3.72 (s, 3H), 3.02-2.90 (m, 1H), 2.79 (dd, *J₁* = 9.0 Hz, *J₂* = 18.0 Hz, 1H), 2.44 (dd, *J*₁ = 6.0 Hz, *J₂* = 15.0 Hz, 1H), 0.82 (s, 9H). ¹³C NMR (75 MHz, CDCl₃) δ 174.9, 77.4, 77.0, 76.6, 51.5, 38.6, 30.0, 29.9, 29.7, 29.0. HRMS (ESI) berechnet für C₇H₁₂O₄Na⁺: 183.06278; gefunden: 183.06277.
**Methylphenylpropanoat⁵ (3g):** ¹H NMR (300 MHz, CDCl₃) δ 7.24-7.18 (m, 3H), 7.15-7.10 (m, 2H), 3.69-3.61 (m, 0.28H), 3.59-3.58 (m, 3H), 2.88 (t, *J* = 9.0 Hz, 1.6H), 2.56 (t, *J* = 9.0 Hz, 1.6H), 1.43 (d, *J* = 6.0 Hz, 0.84H). ¹³C NMR (75 MHz, CDCl₃) δ 177.1, 153.9, 128.7, 127.2, 124.2, 124.1, 110.6, 77.5, 77.1, 76.6, 44.6, 24.3, 10.2. HRMS (ESI) berechnet für C₁₀H₁₂O₂: 164.08318; gefunden: 164.08349.
**3**-**Ethylbenzofuran**-**2(3H)**-**on⁶ (3h):** ¹H NMR (300 MHz, CDCl₃) δ 7.24-7.17 (m, 2H), 7.09-7.00 (m, 2H), 3.61 (t, *J* = 6.0 Hz, 1H), 2.03-1.93 (m, 2H), 0.91-0.86 (m, 3H). ¹³C NMR (75 MHz, CDCl₃) δ 175.0, 173.3, 140.6, 140.5, 128.6, 128.5, 128.3, 127.5, 127.1, 126.3, 77.4, 77.0, 76.6, 51.5, 35.7, 30.9, 18.6. HRMS (ESI) berechnet für C₁₀H₁₀O₂: 162.06753; gefunden: 162.06753.
**Methyl- 2-(2-hydroxyphenyl)butanoat⁷ (3i):** ¹H NMR (300 MHz, CDCl₃) δ 7.13-7.08 (m, 1H), 7.00-6.97 (m, 1H), 6.86-6.76 (m, 2H), 3.68 (s, 3H), 3.51 (t, *J* = 6.0 Hz, 1H), 2.13-1.98 (m, 1H), 1.92-1.78 (m, 1H), 0.83 (t, *J* = 7.5 Hz, 3H). ¹³C NMR (75 MHz, CDCl₃) δ 177.7, 155.1, 130.4, 129.0, 123.8, 120.6, 118.2, 77.5, 77.0, 76.6, 52.7, 51.6, 24.5, 12.3. HRMS (ESI) berechnet für C₁₁H₁₄O₃: 195.10157; gefunden: 195.10173.
**Methyl-3-phenylbutanoat⁸ (3j):** ¹H NMR (300 MHz, CDCl₃) δ 7.25-7.10 (m, 5H), 3.55 (s, 3H), 3.22-3.20 (m, 1 H), 2.60-2.43 (m, 2H), 1.23 (d, *J* = 6 Hz, 3H). ¹³C NMR (75 MHz, CDCl₃) δ 172.9, 145.7, 128.5, 126.7, 126.4, 126.3, 77.5, 77.1, 76.6, 51.5, 42.8, 36.5, 21.8. HRMS (ESI) berechnet für C₁₁H₁₄NaO₂⁺: 201.0886; gefunden: 201.08843.
**Methyl -3-*p*-tolylbutanoat⁸ (3k):** ¹H NMR (300 MHz, CDCl₃) δ 7.04 - 7.03 (m, 4H), 3.55 (s, 3H), 3.19-3.16 (m, 1 H), 2.58-2.41 (m, 2H), 2.24 (s, 3H), 1.21 (d, *J* = 9 Hz, 3H). ¹³C NMR (75 MHz, CDCl₃) δ 172.9, 142.7, 135.9, 129.2, 126.6, 77.5, 77.1, 76.6, 51.5, 42.8, 36.0, 21.9, 21.0. HRMS (ESI) berechnet für C₁₂H₁₆NaO₂⁺: 215.10425; gefunden: 215.10426.
**Methyl 3**-(**4**-**chlorophenyl**)**butanoat⁸ (3l):** ¹H NMR (300 MHz, CDCl₃) δ 7.20-7.17 (m, 2H), 7.09-7.07 (m, 2H), 3.55 (s, 3H), 3.22-3.16 (m, 1H), 2.54-2.43 (m, 2H), 1.20 (d, *J* = 6 Hz, 3H). ¹³C NMR (75 MHz, CDCl₃) δ 172.5, 144.1, 132.1, 128.6, 128.1, 77.4, 77.1, 76.7, 51.6, 42.6, 36.9, 21.8. HRMS (ESI) berechnet für C₁₁H₁₃NaClO₂⁺: 237.04697; gefunden: 237.04701.
**Methyl**-**3**-(**naphthalen**-**2**-**yl**)**butanoat⁸ (3m):** ¹H NMR (300 MHz, CDCl₃) δ 7.73-7.70 (m, 3H), 7.57 (s, 1H), 7.39-7.27 (m, 3H), 3.53 (s, 3H), 3.40- 3.35 (m, 1H), 2.68- 2.53 (m, 2H), 1.31 (d, *J* = 6 Hz, 3H). ¹³C NMR (75 MHz, CDCl₃) δ 172.9, 143.1, 133.6, 132.4, 128.2, 127.7, 127.6, 126.0, 125.5, 125.4, 125.0, 77.4, 77.1, 76.8, 51.6, 42.7, 36.6, 21.8. HRMS (ESI) berechnet für C₁₅H₁₆NaO₂⁺: 251.10425; gefunden: 251.10471.
**Dimethylglutarat⁹ (3n):** ¹H NMR (300 MHz, CDCl₃) δ 3.70 (s, 6H), 2.41 (t, *J* = 7.5 Hz, 4H), 1.98 (tt, *J₁* = 9.0 Hz, *J₂* = 15.0 Hz, 2H). ¹³C NMR (75 MHz, CDCl₃) δ 173.4, 77.5, 77.1, 76.6, 51.6, 33.0, 20.1. HRMS (ESI) berechnet für C₇H₁₂O₄Na⁺: 183.06278; gefunden: 183.06284.
Dimethyl **-1,19-nonadecanedioat¹⁰ (3o):** ¹H NMR (300 MHz, CDCl₃) δ 3.60 (s, 6H), 2.23 (t, *J* = 7.5 Hz, 4H), 1.57-1.52 (m, 4H), 1.26-1.18 (m, 26H). ¹³C NMR (75 MHz, CDCl₃) δ 174.4, 77.5, 77.0, 76.6, 51.5, 34.1, 29.7, 29.6, 29.5, 29.3, 29.2, 25.0. HRMS (ESI) berechnet für C₂₁H₄₀O₄H⁺: 357.29994; gefunden: 357.29979.

### Literatur

(1) Zimmermann, F.; Meux, E.; Mieloszynski, J.-L.; Lecuire, J.-M.; Oget, N. Tetrahedron Letters 2005, 46, 3201.
(2) Vieira, T. O.; Green, M. J.; Alper, H. Organic Letters 2006, 8, 6143.
(3) Fagnoni, M.; Mella, M.; Albini, A. Journal of Organic Chemistry 1998, 63, 4026.
(4) Zhu, B. C.; Jiang, X. Z. Applied Organometallic Chemistry 2006, 20, 277.
(5) Poeylaut-Palena, A. A.; Testero, S. A.; Mata, E. G. Chemical Communications (Cambridge, United Kingdom) 2011, 47, 1565.
(6) Wang, H.; Dong, B.; Wang, Y.; Li, J.; Shi, Y. Organic Letters 2014, 16, 186.
(7) Liu, G., Yves; Hutchinson, J. H.; Therien, M.; Delorme, D.; (Merck Frosst Canada Inc., Can.). Application: WO, 1994, p 147 pp.
(8) Liu, Q.; Wu, L.; Jackstell, R.; Beller, M. ChemCatChem 2014, 6, 2805.
(9) de Meijere, A.; Limbach, M.; Janssen, A.; Lygin, A.; Korotkov, V. S. European Journal of Organic Chemistry 2010, 3665.
(10) Fleischer, I.; Jennerjahn, R.; Cozzula, D.; Jackstell, R.; Franke, R.; Beller, M. ChemSusChem 2013, 6, 417.

## Patentansprüche

1. Verfahren zur Alkoxycarbonylierung von ethylenisch ungesättigten Verbindungen, **dadurch gekennzeichnet, dass** mindestens eine ethylenisch ungesättigte Verbindung mit Formalin oder Paraformaldehyd und einem Alkohol ausgewählt aus Methanol und Isopropanol in Gegenwart eines Palladiumkatalysators, mindestens einem Phosphinliganden und einer Säure umgesetzt wird.

2. Verfahren nach Anspruch 1, **dadurch gekennzeichnet, dass** der Palladiumkatalysator einen Palladiumkomplex mit Palladium in den Oxidationsstufen 0 bis +II umfasst und vorzugsweise ausgewählt ist aus der Gruppe enthaltend Pd-Acetat, Pd-Acetylacetonat, Pd-Halogenide und Pd-Dibenzylidenaceton.

3. Verfahren nach Anspruch 1 oder 2, **dadurch gekennzeichnet, dass** die Phosphinliganden eine bidentate Struktur aufweisen.

4. Verfahren nach Anspruch 3, **dadurch gekennzeichnet, dass** der Phosphinligand 1,2-Bis(di-*tert*-butylphosphinomethyl)benzol (d^{t}bpx) ist.

5. Verfahren nach einem der Ansprüche 1 bis 4, **dadurch gekennzeichnet, dass** die Säure eine Sulfonsäure oder Phosphinsäure ist, vorzugsweise ausgewählt aus Methansulfonsäure und Toluolsulfonsäuren und Diarylphosphinsäuren.

6. Verfahren nach Anspruch 5, **dadurch gekennzeichnet, dass** der Phosphinligand in einer Menge vorliegt, die unterhalb der Menge an Sulfonsäure liegt oder der Menge an Sulfonsäure entspricht.

7. Verfahren nach einem der Ansprüche 1 bis 6, **dadurch gekennzeichnet, dass** die Reaktionstemperatur bei 20 bis 160°C liegt, bevorzugt bei 80 bis 140°C.

8. Verfahren nach einem der Ansprüche 1 bis 7, **dadurch gekennzeichnet, dass** der Druck im Bereich von 1 bis 60 bar liegt, vorzugsweise Normaldruck.

9. Verfahren nach einem der Ansprüche 1 bis 8, **dadurch gekennzeichnet, dass** als ethylenisch ungesättigte Verbindungen einfach oder mehrfach ungesättigte lineare n-Alkene, verzweigte Isoalkene oder Cycloalkene oder aromatische Alkene mit einer Kohlenstoffzahl von 2 bis 40 eingesetzt werden, die gegebenenfalls substituiert sind.

## Claims

1. Method for the alkoxycarbonylation of ethylenically unsaturated compounds, **characterized in that** at least one ethylenically unsaturated compound is reacted with formalin or paraformaldehyde and an alcohol selected from methanol and isopropanol in the presence of a palladium catalyst, at least one phosphine ligand and an acid.

2. Method according to Claim 1, **characterized in that** the palladium catalyst comprises a palladium complex with palladium in the oxidation state 0 to +11 and is preferably selected from the group comprising Pd acetate, Pd acetylacetonate, Pd halide and Pd dibenzylideneacetone.

3. Method according to Claim 1 or 2, **characterized in that** the phosphine ligands have a bidentate structure.

4. Method according to Claim 3, **characterized in that** the phosphine ligand is 1,2-bis(di-tert-butylphosphinomethyl)benzene (d^{t}bpx).

5. Method according to any of Claims 1 to 4, **characterized in that** the acid is a sulphonic acid or phosphinic acid, preferably selected from methanesulphonic acid and toluenesulphonic acids and diarylphosphinic acids.

6. Method according to Claim 5, **characterized in that** the phosphine ligand is present in an amount which is below the amount of sulphonic acid or corresponds to the amount of sulphonic acid.

7. Method according to any of Claims 1 to 6, **characterized in that** the reaction temperature is at 20 to 160°C, preferably at 80 to 140°C.

8. Method according to any of Claims 1 to 7, **characterized in that** the pressure is in the range of 1 to 60 bar, preferably standard pressure.

9. Method according to any of Claims 1 to 8, **characterized in that** the ethylenically unsaturated compounds used are monounsaturated or polyunsaturated linear n-alkenes, branched isoalkenes or cycloalkenes or aromatic alkenes having a carbon number of 2 to 40, which are optionally substituted.

## Revendications

1. Procédé pour l'alcoxycarbonylation de composés éthyléniquement insaturés, **caractérisé en ce qu'**au moins un composé éthyléniquement insaturé est transformé avec de la formaline ou du paraformaldéhyde et un alcool choisi parmi le méthanol et l'isopropanol en présence d'un catalyseur à base de palladium, d'au moins un ligand de type phosphine et d'un acide.

2. Procédé selon la revendication 1, **caractérisé en ce que** le catalyseur à base de palladium comprend un complexe du palladium avec du palladium dans les étages d'oxydation de 0 à +II et est de préférence choisi dans le groupe contenant l'acétate de Pd, l'acétylacétonate de Pd, les halogénures de Pd et le dibenzylidène-acétone de Pd.

3. Procédé selon la revendication 1 ou 2, **caractérisé en ce que** les ligands de type phosphine présentent une structure bidentate.

4. Procédé selon la revendication 3, **caractérisé en ce que** le ligand de type phosphine est le 1,2-bis(di-tert-butylphosphinométhyl)benzène (d^{t}bpx).

5. Procédé selon l'une quelconque des revendications 1 à 4, **caractérisé en ce que** l'acide est un acide sulfonique ou un acide phosphinique, de préférence choisi parmi l'acide méthanesulfonique et les acides toluènesulfoniques et les acides diarylphosphiniques.

6. Procédé selon la revendication 5, **caractérisé en ce que** le ligand de type phosphine se trouve en une quantité qui est inférieure à la quantité d'acide sulfonique ou qui correspond à la quantité d'acide sulfonique.

7. Procédé selon l'une quelconque des revendications 1 à 6, **caractérisé en ce que** la température de réaction est de 20 à 160°C, de préférence de 80 à 140°C.

8. Procédé selon l'une quelconque des revendications 1 à 7, **caractérisé en ce que** la pression se situe dans la plage de 1 à 60 bars, de préférence à la pression normale.

9. Procédé selon l'une quelconque des revendications 1 à 8, **caractérisé en ce qu'**on utilise, comme composés éthyléniquement insaturés, des n-alcènes linéaires, monoinsaturés ou polyinsaturés, des isoalcènes ramifiés ou des cycloalcènes ou des alcènes aromatiques présentant un nombre d'atomes de carbone de 2 à 40, qui sont le cas échéant substitués.
